# EUROPEAN PATENT APPLICATION

(11) **EP 0 561 491 A2**
(43) Date of publication of application: **22.09.1993**
(21) Application number: 93300648.8
(22) Date of filing: 28.01.1993
(51) Int. Cl.: A61N 1/365, A61N 1/39

(54) **Cardioverting/defibrillating device with off-line ECG analysis**

(30) Priority: 16.03.1992 US 851624
(71) Applicant: TELECTRONICS N.V., Curaçao (AN)
(72) Inventor: Somerville, William M., Aspley, Q.L.D. 4034 (AU); Wickham, John P., Five Dock, N.S.W. 2046 (AU)
(74) Representative: Hackett, Sean James

(57) **Abstract**

An improved implantable pacemaker/defibrillator device in which a first and relatively simplistic technique is employed to detect an abnormal heart rhythm and a second more precise algorithm is utilized for the more difficult problem of confirming the arrythmia. Since the complex, higher power consuming device is utilized only for confirmation, overall power consumption is minimized. Optionally, the precise algorithm may be utilized periodically to adjust the sensitivity of the detection circuitry.

## Description

### Technical Field of the Invention

This invention relates to arrythmia reverting devices and, more particularly, to an improved method and apparatus for detecting an arrhythmia and providing electrical therapy to shock the heart back to normal sinus rhythm.

### Description of the Prior Art

Many advances have recently been made in implantable pacemaker/defibrillator devices which allow these devices to recognize an abnormally functioning heart and to provide therapy in the form of one or more electrical discharges into the heart in order to shock the heart back to normal sinus rhythm. One example of such a device is disclosed in U. S. patent No. 3,952,750 issued to Mirowski, et al.

Despite such advances, there is still room for improvement in this field. In particular, the device must be capable of accurately identifying the presence of an abnormal heart rhythm; e.g., ventricular tachyrhythmia, ventricular fibrillation, etc. Moreover, not only is it imperative that the device recognize the presence of the arrhythmia accurately, but it is essential that the arrhythmia be confirmed prior to aggressive electrical therapy being delivered to the heart. Specifically, there is normally a period of time of up to 30 seconds from the time a ventricular tachyrhythmia or fibrillation is detected until the shock is delivered to the heart. The arrythmia could, for example, spontaneously revert during this time. The presence of the arrythmia must therefore be accurately confirmed at the end of this period of time, just prior to delivery of the electrical therapy.

Although it is difficult to detect and confirm arrhythmias, the confirmation step is particularly troublesome when compared with initial detection. This is because large random variations in the magnitude of the ECG signal occur during the time period between detection and confirmation. If the amplitude of the ECG signal produced by the abnormal heartbeat decreases by too much, the device may determine that the arrythmia has reverted when it really hasn't. Conversely, if the ECG amplitude of the normal heartbeat increases even though the arrythmia reverts, the device will over-sense and confirm the presence of an arrythmia when, in fact, the arrythmia is no longer present.

Additionally, even in the initial detection step, problems with changing body resistance, lead characteristics, etc., hinder accurate measurements of ECG signals. Any error in the detection or confirmation of an abnormal heart rhythm results in either unnecessary electrical shock being delivered to the heart, or electrical therapy not being delivered when such therapy is needed.

It can be appreciated from the above that the need to accurately detect and confirm arrhythmias requires accurate and adaptive sensing and measurement of ECG signals. Indeed, the Williams et al. article, entitled "Automatic Implantable Cardioverter-Defibrillator-Related Complications," Journal of the American College of Cardiology, Vol. 15, No. 2, Abstracts, P. 55A, February 1990, reports that .6% of deaths associated with implantable cardioverter defibrillators were due to sensing failure, and that 4.9% of non-fatal complications were due to over-sensing. More simply put, the implantable device must be capable of (1) accurately detecting an arrythmia even though the measured ECG signal resulting from such an arrythmia may be larger or smaller than the ECG signal from a previous arrythmia; and (2) accurately confirming an arrythmia even though the ECG signal may greatly and rapidly vary in amplitude from the time of detection until confirmation.

One technique for addressing the second of the above problems is described in U. S. Patent No. 4,940,054, entitled "Apparatus and Method for Controlling Multiple Sensitivities in an Antitachyarrhythmia Device", to Richard Grevis and Norma Gilli. In this device, the gain of the sensing system is increased prior to the confirmation point. This increase in gain increases the probability of confirmation. A potential problem with this arrangement is that if spontaneous reversion occurs after detection and prior to confirmation, but the normal sinus rhythm produces a signal which is slightly greater than normally expected, the detector may confirm the arrhythmia, even though such arrhythmia is no longer present. Such false confirmation results in unnecessary, and indeed possibly harmful, electrical shocks being delivered to the heart.

An additional prior art system addressed to the above problems is described in U. S. Patent No. 4,819,643 to J. Menken. The Menken arrangement provides for automatic gain control of the signal being utilized to detect and confirm an arrhythmia. As the amplitude of the ECG signal varies, the gain of an amplifier detecting the ECG signal varies inversely. Thus, decreases in ECG amplitude are compensated for by increases in gain. However, even the Menken system does not fully solve the problem, as explained by Bardy et al. in the article, "Failure of the Automatic Implantable Defibrillator to Detect Ventricular Fibrillation", American Journal of Cardiology, Vol. 58, November 15, 1986, pp. 1107-1108. Specifically, even though automatic gain control is present in the device, the gain cannot be adjusted fast enough to follow rapid fluctuations in the amplitude of electrocardiac responses which occur between detection and confirmation. Such rapid fluctuations are often present, for example, during ventricular fibrillation (VF). Indeed, Bardy et al. conclude the article by stating that there is a "limitation of an arrhythmia-sensing algorithm that depends on automatically adjusting the gain of an electrical signal to detect VF while that signal may be changing rapidly in magnitude".

In addition to the above systems, there are prior arrangements that have attempted to utilize digital storage techniques in conjunction with implantable pacing systems, although none are directed to the sensitivity adjustment problem. U.S. Patent No. 4,716,903 to Hansen et al. ("Storage in a Device Memory") discloses an implantable device which utilizes digital storage and a compression algorithm so that a representation of an ECG signal may be stored and analyzed at a later time by a physician. Australian patent No. 8823170, issued to H. Lagergren and entitled "Pacemaker System", teaches the storage of an ECG signal on a rolling basis so that a finite window of samples is always available in memory. Finally, U.S. Patent 4,913,146 issued to R. DeCote, Jr. ("Cardiac Sense Amplifier with Pattern Recognition Capabilities") discloses a device which processes a filtered ECG signal by utilizing a microprocessor. From the processed and filtered signal, a set of cardiac waveform descripters is derived. These waveform descripters may then be stored in memory as a template. When another template taken at a later time differs from the stored template by a sufficient amount, it is determined that an arrhythmia or other abnormality is present.

### Summary of the Invention

The above problems of the prior art are overcome and numerous other advantages achieved in accordance with the present invention which relates to an improved apparatus for processing stored samples of an electrocardiogram (ECG) signal to more accurately detect and reconfirm the presence of an arrhythmia. Specifically, upon detection of an arrythmia by a relatively simple detection means, data samples representing a portion of an ECG signal are stored in random access memory (RAM). The portion of the ECG signal stored is large enough to contain several R-waves, i.e., at least two. A second and more precise algorithm operates on the stored samples utilizing advanced signal processing techniques which are well known in the art. The second algorithm is used to confirm the arrythmia just prior to delivering electrical shock to the heart. By utilizing a conventional technique to detect the arrythmia, power is conserved. The more precise and complex confirmation algorithm, which requires higher power consumption, is utilized only to confirm the arrythmia after detection, thereby minimizing overall power consumption.

In another embodiment, the signal processing algorithm may also be utilized to adjust the sensitivity of the arrythmia detector. For example, once a day, the algorithm can be utilized to count heartbeats for a period of time while the arrythmia detector does the same. If the arrythmia detector detects fewer heartbeats than the algorithm, the sensitivity of the arrythmia detector is increased. Conversely, the sensitivity of the arrythmia detector is decreased if it detects more heartbeats than the signal processing algorithm. Thus, the algorithm is utilized to improve operation of the arrythmia detector.

Advantageously, the confirmation algorithm is more precise than the conventional detection means in that the algorithm can better adjust for rapid signal amplitude variations, variations in lead resistance, etc. By storing a digital representation of a plurality of R-waves and performing a detailed analysis, not necessarily in real-time , a much more accurate determination of ECG activity can be obtained than that obtained from a conventional detection technique.

### Brief Description of the Drawings

FIG. 1 is a block diagram of a microprocessor based system for implementing the method of the present invention;
FIG. 2 is an exemplary flow diagram showing the major steps of an algorithm for implementing the present invention; and
FIG. 3 is an additional flow diagram showing other portions of a preferred algorithm.

### Detailed Description of the Preferred Embodiments

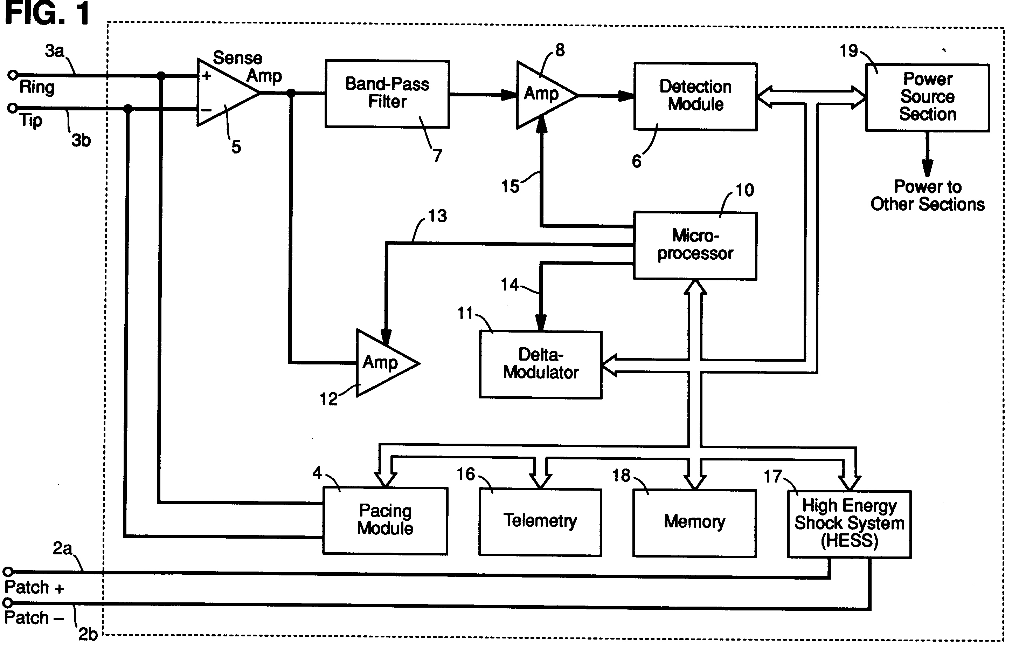

FIG. 1 is a block diagram of an implantable pacing device in accordance with the invention. The device includes numerous amplifiers, detection modules, and other electronic components to be described hereafter. Additionally, the pacing device includes a high-energy shock system (HESS) 17 for delivering defibrillating shocks to the heart when necessary.

Sensing amplifier 5 receives signals from conductors 3a and 3b, and amplifies the difference therebetween. This difference is processed by band-pass filter 7 to remove slow drifts and attenuate the amplitude of T waves in the signal from the sensing and pacing leads. The band-pass-filtered signal is then supplied to selectable gain amplifier 8, the gain of which is controlled by microprocessor 10 in accordance with the result dictated by a signal processing algorithm.

Amplifier 12 is also arranged to receive signals from conductors 3a and 3b through sensing amplifier 5. Amplifier 12 provides the amplified signal to delta modulator 11 which samples the signal and provides a plurality of digital samples to microprocessor 10. A finite window of samples is provided for use by microprocessor 10 in the signal processing algorithm for determining the proper gain adjustments to be made to amplifier 8 and for detecting the arrythmia. Delta modulator 11 is also utilized to provide the samples to be processed for confirmation, as described below.

The pacing system also includes conventional elements such as a pacing module 4, working memory 18, and a power source 19. Telemetry means 16 is any of a variety of well-known arrangements of circuitry for telemetrically communicating with a physician's equipment located external to the patient's body. Finally, high-energy shock system (HESS) 17 may be invoked as needed in order to provide defibrillating electrical shocks to the cardiac tissue via leads 2a and 2b if the pacing device determines that such shocks are needed.

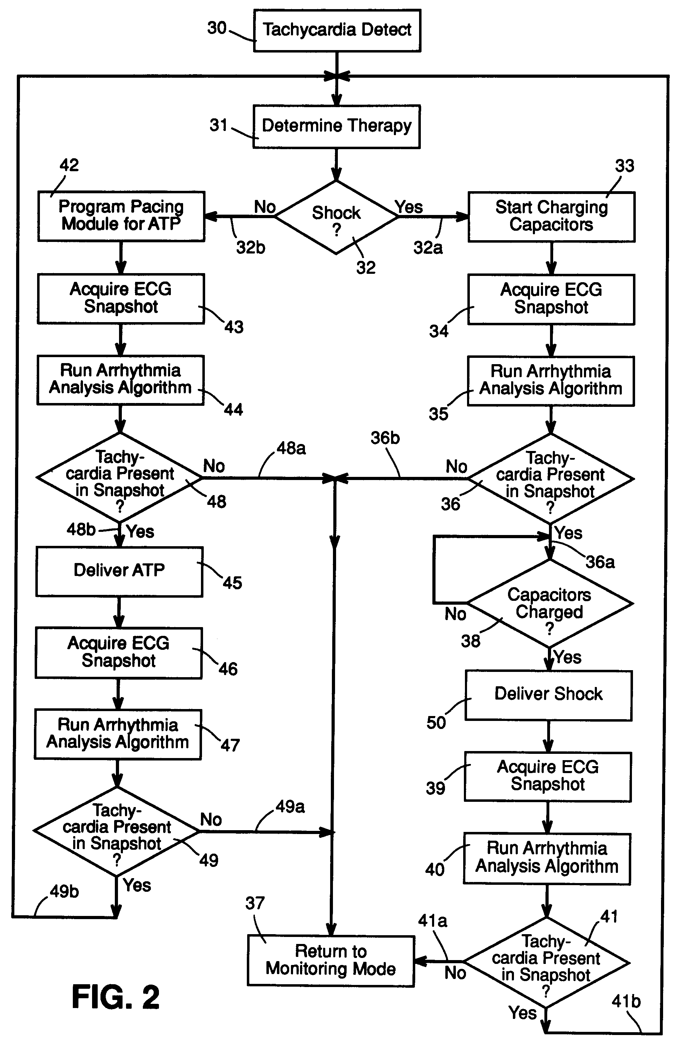

Turning now to FIG. 2, shown therein is a flow chart which may be used in programming microprocessor 10 in order to implement the invention. The following description of FIG. 2 discusses how the algorithm is utilized to confirm the arrythmia.

During normal sinus rhythm, the system is in a monitoring state. The delta modulator 11 is not sampling ECG signals, and the shock capacitors included in the HESS are not charged. The microprocessor may optimally be put in a standby mode during this time so that power consumption may be significantly reduced. The flow chart in FIG. 2 is entered upon detection of an arrhythmia at block 30. The proper therapy required to end the arrhythmia and revert the heart to normal sinus rhythm is determined in accordance with a therapy strategy algorithm at block 31. For example, the therapy strategy algorithm may determine the parameters for the train of pacing pulses to be delivered to the heart, or the required energy for reverting the arrhythmia. Proper determination of the therapy to be delivered depends upon, among other things, the nature of the arrhythmia detected by detection module 6 of FIG. 1. A variety of well-known therapy strategies and algorithms are presently in use; the particular algorithm utilized may vary from system to system.

If the therapy strategy algorithm determines at decision point 32 that a defibrillating shock should be delivered to the heart, block 33 is entered via branch 32a and the defibrillation capacitors begin to charge. As the defibrillation capacitors begin to charge, the delta modulator 11 is activated and begins providing samples of electrocardiac activity for a predetermined time window. The time window is long enough to include multiple R-waves, and is represented in FIG. 2 by block 34, labelled "acquire ECG snapshot".

After an ECG snapshot is acquired, i.e., the predetermined time window has elapsed, all samples from delta modulator 11 are input to the arrhythmia analysis algorithm and processed accordingly as indicated in block 35. There are two main parts to the arrhythmia analysis algorithm. The first part extracts significant events from the sampled signal, as may be done utilizing the method given in copending U.S. Patent Application Serial No. 851,524, filed March 16, 1992. It is emphasized, however, that any method of extracting events may be utilized without violating the scope of the invention.

The second part of the algorithm is utilized to determine the present cardiac rhythm. This may be done utilizing a variety of straightforward techniques. For example, if N events are detected as represented by the samples in the window of time of the ECG snapshot and the snapshot is Y units wide, it can be determined that an event is occurring every Y/N time units. As cardiac rhythm increases toward tachyrhythmia and ultimately to VF, Y/N becomes smaller. Accordingly, it can be determined whether particular arrhythmias are present by comparing Y/N to a predetermined set of thresholds.

It is preferable that the ECG snapshot be acquired and analyzed at such time that the analysis is completed just prior to completion of the charging of the shock capacitors. For example, if the shock capacitors take 30 seconds to charge and the ECG snapshot takes 4 seconds to acquire and 10 seconds to analyze, then the system should begin acquiring the ECG snapshot just a little less than 16 seconds after the arrythmia is detected. In that case, the 14 seconds for acquisition and analysis will conclude 30 seconds after detection. Since the shock capacitors begin charging immediately after detection and take 30 seconds to charge, the system will be fully charged just when the arrythmia is confirmed.

After the arrhythmia analysis algorithm is run, a decision is made at decision point 36 as to whether or not an arrhythmia is present in the analyzed ECG snapshot. For example, the arrhythmia analysis algorithm could determine that ventricular tachycardia is present, ventricular fibrillation, etc.

If the arrhythmia analysis algorithm determines that normal sinus rhythm is present in the ECG snapshot, the tachyrhythmia is deemed to have spontaneously reverted and processing proceeds along decision 36b and returns to the monitoring mode in which the system existed prior to the detection of an arrhythmia, as shown at block 37. Optionally, the episode may be logged in memory for later analysis by the physician who may read the data from memory telemetrically. Furthermore, prior to returning to monitoring mode, any charge on the shock capacitors is dissipated, the HESS is placed in the off state, and all elements return to a low power consumption mode.

If the presence of tachyrhythmia is confirmed at decision point 36, processing proceeds along 36a to decision point 38. Decision point 38 is, in effect, a simple programming loop which continues to cycle around upon itself thereby allowing time for the shock capacitors to charge. After such charge has occurred, control is transferred to block 50 which delivers a shock to the heart in an attempt to revert the tachyrhythmia. After the shock is delivered, control is transferred to block 39 where the processor acquires another ECG snapshot. After this snapshot is acquired, the arrhythmia analysis algorithm is repeated at block 40 in order to determine whether the tachyrhythmia has reverted. Decision point 41 then transfers control to monitoring mode block 37 via branch 41a if the tachyrhythmia has reverted. The steps executed in monitoring mode 37 are the same as those previously described, e.g., lower power consumption, etc. If the analysis algorithm run at block 40 indicates that the shock delivered at block 50 was not successful in reverting the tachyrhythmia, then decision point 41 transfers control back up via branch 41b to block 31 and the process of formulating a therapy strategy thereby repeats.

Having examined the sequence of steps executed when it is determined at decision point 32 that a shock should be delivered to the heart, the following discussion addresses the sequence of steps executed when it is determined that no shock should be delivered but, rather, that antitachyarrhythmia pacing (ATP) should be administered instead. In this situation, branch 32b from decision point 32 is taken, and the proper ATP parameters are programmed into pacing module 4 of FIG. 1 as shown at block 42. Before delivering ATP to the heart, blocks 43 and 44 acquire and analyze an ECG snapshot for the purpose of reconfirming the presence of the tachyrhythmia. Decision point 48 returns the system to the monitoring mode via branch 48a and block 37 if the tachyrhythmia is no longer present, i.e., spontaneous reversion has occurred.

If decision point 48 confirms the tachyrhythmia, branch 48b is taken and control is transferred to block 45 in order to deliver ATP. Blocks 46 and 47 then serve to determine whether or not the arrhythmia is still present, as previously described for block 39 and 40. Finally, decision point 49 returns the system to the monitoring mode via branch 49a and block 37 if the ATP has been successful in reverting the arrhythmia, but transfers control to block 31 via branch 49b for re-administering therapy if the ATP has been unsuccessful.

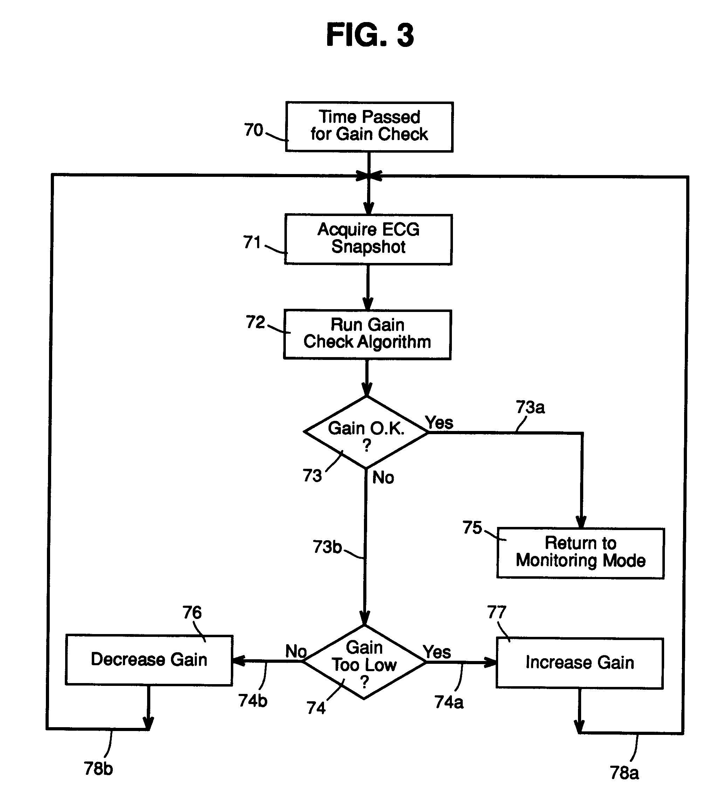

FIG. 3 is a flow diagram of a gain adjustment algorithm which is utilized to adjust the gain of selectable gain amplifier 8 of FIG. 1 or, equivalently, the sensitivity of the arrythmia detector 6. The algorithm shown by the flow chart in FIG. 3 is run at regular infrequent intervals, e.g., once a day or less, and is used to allow the non-real-time signal processing to assist in adjusting subsequent initial detection of an arrythmia. Of course, the actual interval used could be selected by the physician and even varied using telemetric means.

When a timer (not shown) indicates that the proper amount of time has passed for a gain adjustment process to occur, the system will first check to assure that no type of therapy, such as that described with reference to FIG. 2, is currently being delivered. Thus, the gain adjustment process can only be invoked from the monitoring mode. The gain adjustment process is entered at block 70 of FIG. 3 by a signal from the timer. Control is transferred to block 71 which acquires an ECG snapshot in the manner previously described. The delta modulator used to acquire the ECG snapshot for gain adjustment of amplifier 8 may be the same delta modulator as previously described. After an ECG snapshot is acquired, control is transferred to block 72 in order to run a gain check algorithm to determine whether the gain in amplifier 8 must be increased or decreased. One example of a gain check algorithm, although quite simplistic, is to simply count the number of heartbeats detected by the detection module 6. If the therapy strategy algorithm detects many more heartbeats than the detection module 6, it is assumed that the gain of the detection module 6 is too low. If there are many more detects from the detection module, a decision is made that the gain of the detection module is too high.

The algorithm used to extract events from the snapshot for the purpose of gain verification may be the same algorithm used for arrhythmia analysis. If decision point 73 determines the gain of amplifier 8 is acceptable, it transfers control to block 75 via branch 73a and the system returns to the monitoring mode. If, however, the gain is not correct, control is transferred to decision point 74 via branch 73b. Decision point 74 then increases or decreases the gain accordingly.

The gain of amplifier 8 may be increased or decreased in fixed steps. Each time it is determined that the gain should be raised or lowered, the gain is increased or decreased by one step. The steps are made small enough so as to avoid an unstable system which oscillates about the target gain point but which never stabilizes.

It is noted that the details of spectrum analysis, digital signal processing, and other related fields have not been described in detail herein. However, any of the multitude of algorithms available may be utilized in analyzing and extracting information from an ECG snapshot. For example, the original signal from which the delta values were derived could be reconstructed and a correlation used to match the resulting ECG to a template. A template could be one of a library of templates corresponding to different rhythms which may have similar rates but which respond most favorably to different electrotherapies. This would allow different therapies to be delivered for arrhythmias which have similar rates but different shapes. Other techniques may be used on the ECG snapshots, such as Fourier analysis, noise filtering, etc. The well-known text, Digital Signal Processing, by Oppenheim and Schafer (Prentice Hall, Englewood Cliffs, N.J.), describes a variety of still other signal processing techniques which may be utilized with the present invention. Not only can the signals be processed in non-real-time, but they may also be processed out of order.

## Claims

1. Apparatus for administering electrotherapy to a patient's heart to restore a normal heart rhythm comprising:
means for detecting an abnormality in the heart rhythm;
means, responsive to said detecting means detecting a rhythm abnormality, for storing a digital representation of at least a portion of an electrocardiac signal;
means for processing said stored digital representation to confirm said rhythm abnormality, said processing means being operative to confirm a rhythm abnormality with a degree of precision significantly higher than the degree of precision with which said detecting means operates; and
means for delivering appropriate electrocardiac therapy in response to said processing means confirming said rhythmic abnormality.

2. Apparatus according to claim 1 wherein said processing means employs digital signal processing.

3. Apparatus according to claim 1 further including an amplifier having an adjustable gain for amplifying the patient's electrocardiac signal, said amplifier having an output coupled to said detecting means.

4. Apparatus according to claim 3 further including means for periodically comparing a number of cardiac events detected by said detecting means during a predetermined time period to a number of cardiac events detected by said processing means during the predetermined time period, and means for adjusting the gain of said amplifier in accordance with the results of the comparison.

5. Apparatus according to claim 1 further including means for periodically comparing electrocardiac activity detected by said detecting means with electrocardiac activity processed by said processing means; and
means for adjusting said detecting means in response thereto.

6. A method of administering electrotherapy to a patient's heart to restore a normal heart rhythm comprising the steps of:
detecting an abnormality in the heart rhythm;
storing, in response to said step of detecting, a digital representation of at least a portion of an electrocardiac signal;
processing said stored digital representation to confirm said rhythm abnormality with a degree of precision significantly higher than the degree of precision of said detecting step; and
delivering appropriate electrocardiac therapy.

7. The method according to claim 6 wherein said step of processing includes digital signal processing.

8. The method according to claim 6 further including the step of amplifying said electrocardiac signal with an amplifier having an adjustable gain.

9. The method according to claim 8 further including the steps of periodically comparing the number of detected cardiac events to the number of processed cardiac events during a predetermined time period, and adjusting the gain of said amplifier in accordance with the results of the comparison.

10. The method according to claim 6 further including periodically comparing electrocardiac activity detected by said detecting step with electrocardiac activity detected by said processing step and;
adjusting said detecting step in response thereto.

11. Apparatus for processing an ECG signal produced by a patient's heart comprising:
means for detecting an abnormality in the heart rhythm;
means responsive to said detecting means detecting a rhythm abnormality for storing a digital representation of at least a portion of an electrocardiac signal, said portion being sufficiently large to contain at least two R-waves; and
means for processing said stored digital representation in non-real time to confirm said rhythm abnormality.

12. A method of processing an ECG signal produced by a patient's heart comprising the steps of:
detecting an abnormality in the heart rhythm;
storing, in response to said step of detecting step, a digital representation of at least a portion of an electrocardiac signal, said portion being large enough to include at least two R-waves; and
processing said stored digital representation to confirm said rhythm abnormality only after the entire digital representation has been stored.

13. A method according to claim 12 further including the step of delaying said storing and processing steps sufficiently such that said processing step is completed at approximately the time when it is appropriate to shock the patient's heart in order to revert the abnormality.
